# EUROPÄISCHE PATENTSCHRIFT

(11) **EP 1 880 989 B1**
(45) Veröffentlichungstag und Bekanntmachung des Hinweises auf die Patenterteilung: **11.11.2009**
(21) Anmeldenummer: 07013547.0
(22) Anmeldetag: 11.07.2007
(51) Int. Cl.: C07C 205/06, C07C 201/08

(54) **Verfahren zur Herstellung von Dinitrotoluol**
Method for manufacturing dinitrotoluol
Procédé destiné à la fabrication de dinitrotoluol

(30) Priorität: 21.07.2006 DE 102006033722
(43) Veröffentlichungstag der Anmeldung: 23.01.2008
(73) Patentinhaber: Bayer MaterialScience AG, 51368 Leverkusen (DE)
(72) Erfinder: Pohl, Fritz, Dr., 25541 Brunsbüttel (DE); Lorenz, Wolfgang, Dr., 41540 Dormagen (DE); Muennig, Juergen, Shanghai (CN); Pennemann, Bernd, Dr., 51467 Bergisch Gladbach (DE); Wiechers, Gerhard, Dr., 51381 Leverkusen (DE)

(56) Entgegenhaltungen:
- EP-A1- 0 155 586
- EP-A2- 0 696 571
- WO-A-2005/075407

## Beschreibung

Die Erfindung betrifft ein Verfahren zur Herstellung von Dinitrotoluol durch Nitrierung von Toloul mit Nitriersäure (Gemisch von Salpetersäure und Schwefelsäure), bei dem in einer ersten Stufe das Toluol zu Mononitrotoluol (MNT), nachfolgend das Mononitrotoluol in einer zweiten Stufe zu Dinitrotoluol (DNT) umgesetzt wird.

Dinitrotoluol (DNT) ist ein Zwischenprodukt für die Herstellung von Toluylendiisocyanat (TDI), das ein wichtiges, im großtechnischen Maßstab hergestelltes Vorprodukt zur Herstellung von Polyurethanen ist.

Die Herstellung des Dinitrotoluols durch Nitrierung von Toluol mit Nitriersäure (Gemisch von Salpetersäure und Schwefelsäure) ist bekannt und vielfach beschrieben (Ullmann's Enzyklopedie der technischen Chemie, 4.Auflage, Band 17, Seite 391 ff, 1979, Verlag Chemie Weinheim /New York). Die industrielle Herstellung erfolgt, wie beispielsweise in H.Hermann, J.Gebauer, P. Konieczny, "Industrial Nitration of Toluene to Dinitrotoluene" in ACS-Symposium, Series 623, 234-249, 1996, ed. L.F.Albright, R.V.C Carr, R.J. Schmitt beschrieben, vorwiegend isotherm mit Salpetersäure in Gegenwart von Schwefelsäure als Katalysator kontinuierlich in zwei Stufen derart, dass
- a) das in der Dinitrierung (Nitrierung von MNT zu DNT) erhaltene Reaktionsgemisch durch Phasentrennung getrennt wird und die dabei erhaltene Abfallsäure ("Used Acid") mit Salpetersäure wieder aufgestärkt wird und anschließend mit Toluol gemischt wird und der Mononitrierung (Nitrierung von Toluol zu MNT) zugeführt wird, und
- b) das Reaktionsgemisch der Mononitrierung nach erfolgter Umsetzung in einer Separationsstufe in eine das Mononitrotoluol enthaltende organische und eine vorwiegend die Schwefelsäure enthaltende wässrige Phase ( "Abfallsäure" /"Spent Acid" ) aufgetrennt wird, und
- c) die in b) gewonnene, das Mononitrotoluol enthaltende organische Phase der Dinitrierung zugeführt und dort das Mononitrotoluol mit Salpetersäure in Gegenwart von Schwefelsäure zu Dinitrotoluol umgesetzt wird.

Üblicherweise wird zur Erzielung handelsüblicher Spezifikationen das so gewonnene Roh-DNT in nachgeschalteten Stufen, vorwiegend Wäschen, aufbereitet und so weitgehend von gelösten Schwefel- und Salpetersäuregehalten wie auch von in den Reaktionsstufen gebildeten Nebenkomponenten, z.B. Kresolen und deren Abbauprodukten, befreit. Typische handelsübliche DNT-Produkte weisen DNT-Gehalte > 98,5 Gew.-%, weniger als 0,1 Gew.-% Mononitrotoluol, weniger als 0,1 Gew.-% Trinitrotoluol und weniger als 0,1 Gew.-% sonstiger Nebenkomponenten, bezogen auf das Gewicht der DNT-Produkt Gemisches, bei DNT-Ausbeuten von > 98 % sowie Toluolumsätzen von > 99,9 % auf. Wesentlich ist ebenfalls das Gewichts-Verhältnis der 2,4- und 2,6-DNT-Isomeren in der Summe zu den 2,3-, 3,4-, 2,5 und 3,5-DNT-Isomeren in der Summe. Handelsüblich spezifikationsgerecht liegt der Gehalt der 2,4- und 2,6- DNT-Isomeren in der Summe im Roh-DNT bei > 95 Gew.-%, bezogen auf das Gewicht des Roh-DNT. Bevorzugt beträgt dabei der Gehalt des 2,4-DNT 79,0 - 81,0 Gew.-%, bezogen auf die Summe der Gewichte des 2,4-DNT und des 2,6-DNT. Entsprechend beträgt der Gehalt des 2,6-DNT 19,0 - 21,0 Gew.-%, bezogen auf die Summe der Gewichte des 2,4-DNT und des 2,6-DNT.

Neben dem Roh-DNT wird in dem Verfahren in der Auftrennung des in der Mononitrierung erhaltenen Reaktionsgemischs Abfallsäure gewonnenen, die als zweiter Massenstrom das System verlässt. Die Abfallsäure weist üblicherweise einen Schwefelsäuregehalt von 70 - 74 Gew.-% auf und enthält üblicherweise > 0,1, bevorzugt > 0,2 bis 1,5 Gew.- % an nicht umgesetzter Salpetersäure, Nitrose aus in Nebenreaktionen ablaufenden Oxidationsvorgängen, > 0,2 Gew.-% an MNT, das in der Phasentrennung nicht abgeschieden worden ist, sowie üblicherweise Wasser in einem Konzentrationsbereich von >26 bis < 30 Gew.-% (umfassend das mit der frisch in das Verfahren eingesetzten Schwefelsäure eingetragene, in der Salpetersäure enthaltene sowie das bei der Nitrierungen des Toluols und des Mononitrotoluos entstehende Wasser), jeweils bezogen auf das Gewicht der Abfallsäure.

Bei der zweistufigen isothermen Nitrierung von Toluol zu Dinitrotoluol mit Salpetersäure in Gegenwart von Schwefelsäure ist sowohl der Einsatz hochkonzentrierter Salpetersäure (DE 10 2004 005 913 A) wie auch der Einsatz azeotroper und subazeotroper Salpetersäure (EP 0 903 336 A2) in einem Konzentrationsbereich von > 57 bis 69 Gew.-% beschrieben. Konzentrationen > 69 Gew.-% erfordern einen erhöhten, kostenintensiven technischen Aufwand, der im Wesentlichen durch die Überwindung des Azeotrops verursacht ist.

Die Konzentration der eingesetzten Schwefelsäure, die als Katalysator und als Wasser entziehendes Mittel wirkt, wird in technischen DNT-Anlagen wesentlich von der Konzentration der in das Verfahren eingesetzten Salpetersäure sowie den Reaktionsverhältnissen in der Mononitrierstufe beeinflusst.

Es ist bekannt, dass Salpetersäure unter geeigneten Bedingungen die bei der isothermen zweistufigen Nitrierung von Toluol zu Dinitrotoluol mit Salpetersäure in Gegenwart von Schwefelsäure gehandhabten organische Verbindungen nicht nur zu nitrieren sondern auch zu oxidieren vermag. Mögliche, durch die Oxidation von Toluol, MNT oder DNT erhaltene Verbindungen sind Kresole, Phenole und deren Nitrierprodukte. Eine geringe Schwefelsäurekonzentration verstärkt die Oxidationsneigung der Salpetersäure, so dass der Gehalt an organischen Nebenprodukten im Reaktionsgemisch mit abnehmender Schwefelsäurekonzentration ansteigt. Zum anderen sinkt mit fallender Schwefelsäurekonzentration die Geschwindigkeit der in der Mononitrierung angestrebten Nitrierreaktionen. Beide Phänomene führen bei technischen DNT-Anlagen zu einer Untergrenze der Schwefelsäurekonzentration, bei der dieses Verfahren wirtschaftlich ausgeübt werden kann. Üblicherweise liegt diese Grenze bei etwa 70 Gew.-% (EP 0 903 336 A2).

Dieser Grenzwert hat Auswirkungen auf die Schwefelsäurekonzentration der in der Phasentrennung der Dinitrierstufe gewonnenen und auf die Mononitrierstufe zurückgeführten Absäure. Die Konzentration an H₂SO₄ in der Absäure aus der Dinitrierstufe ist üblicherweise größer als 80 Gew.-%, zum einen um die Schwefelsäuredurchsätze durch die Mononitrierung zu begrenzen und zum anderen um eine hohe Reaktionsgeschwindigkeit der Nitrierung in der Dinitrierstufe zu gewährleisten. Denn da die Konzentration der Schwefelsäure auch auf der Dinitrierstufe die Geschwindigkeit der angestrebten Umsetzungen stark beeinflusst, führen sinkende Konzentrationen zu geringeren Reaktionsgeschwindigkeiten.

Um den skizzierten Konzentrationsanforderungen sowie den oben aufgeführten Spezifikationen eines technischen DNTs zu genügen, werden in technischen DNT-Anlagen nach dem isothermen zweistufigen Verfahren üblicherweise Schwefelsäuren mit Konzentrationen von 93 - 98 Gew.-% H₂SO₄ oder höher, bezogen auf das Gewicht der Schwefelsäure, eingesetzt. Bei Verwendung subazeotroper Salpetersäuren im Bereich von 60 - 65 Gew.-% HNO₃, bezogen auf das Gewicht der Salpetersäure, werden in der Regel Schwefelsäuren mit Konzentrationen von größer 95 Gew.-% H₂SO₄, bezogen auf das Gewicht der Schwefelsäure, eingesetzt. Davon abweichend lehrt EP 0 903 336 A2 den Einsatz 86 - 91 Gew.-%-iger, bevorzugt 87-89 Gew.-%-iger Schwefelsäure. Die eingesetzten Säuren können entweder frisch hergestellt oder durch Aufkonzentrieren der Abfallsäure aus der Phasentrennung des Reaktionsgemischs der Mononitrierung in einer Aufkonzentrieranlage gewonnen werden.

Neben diesem Standardverfahren der 2-stufigen kontinuierlichen isothermen Nitrierung von Toluol mit Salpetersäure in Gegenwart von Schwefelsäure wurde auch vorgeschlagen, die Nitrierung von Toluol zu Dinitrotoluol mit Nitriersäure in drei Stufen kontinuierlich durchzuführen (EP 903 336 A) oder adiabatisch einstufig oder zweistufig so durchzuführen, dass, wie in EP 597 361 A und EP 696 570 A beschrieben, die gesamte Reaktionswärme aus der Nitrierung des Toluols zu DNT oder nur aus der DNT-Stufe, wie in EP 696 571 beschrieben, zum Abtrennen des Reaktionswassers aus der Nitrierung und des durch die Salpetersäure in die Abfallsäure eingebrachten Wassers ausgenutzt wird. Außerdem wurde in US 5 948 944 A und US 2 362 743 A vorgeschlagen, die Nitrierung von Toluol zu DNT nur in Salpetersäure als Reaktionsmedium durchzuführen und so den Einsatz von Schwefelsäure zu vermeiden.

Bei allen Verfahren zur Herstellung von DNT durch Nitrierung von Toluol mit Salpetersäure ist für eine wirtschaftliche Verfahrensführung Voraussetzung, dass die bei den Verfahren anfallenden Absäuren so wieder aufbereitet werden können, dass sie erneut als Reaktionsmedium in den Reaktionsprozess eingesetzt werden können, wie beispielsweise in EP 155 586 A und US 5 275 701 A beschrieben.

Wesentlich für die Auswahl eines Nitrierverfahrens sind jedoch zusätzlich auch seine verfahrensinherente Sicherheit, die Robustheit, mit der es betrieben werden kann, die Selektivität und Vollständigkeit, mit der das Toluol zu Dinitrotoluol umgesetzt werden kann, sowie der spezifische Einsatz an Salpetersäure, der für die Umsetzung des Toluols zu Dinitrotoluol erforderlich ist.

Aufgrund der aufgeführten Kriterien erfolgt die großtechnische Herstellung des Dinitrotoluols aus Toluol mit Salpetersäure vorwiegend nach dem sog. Nitriersäure- oder Mischsäureverfahren, bei dem das Toluol mit Salpetersäure in Gegenwart von Schwefelsäure kontinuierlich in zwei isotherm betriebenen Reaktionsstufen zum Dinitrotoluol umgesetzt wird, wobei
- a) das in der Dinitrierung (Nitrierung von MNT zu DNT) erhaltene Reaktionsgemisch durch Phasentrennung getrennt wird und die dabei erhaltene Abfallsäure ("Used Acid") mit Salpetersäure wieder aufgestärkt wird und anschließend mit Toluol gemischt wird und der Mononitrierung (Nitrierung von Toluol zu MNT) zugeführt wird, und
- b) das Reaktionsgemisch der Mononitrierung nach erfolgter Umsetzung in einer Separationsstufe in eine das Mononitrotoluol enthaltende organische und eine vorwiegend die Schwefelsäure enthaltende wässrige Phase ("Abfallsäure"/"Spent Acid") aufgetrennt wird, und
- c) die in b) gewonnene, das Mononitrotoluol enthaltende organische Phase der Dinitrierung zugeführt und dort das Mononitrotoluol mit Salpetersäure in Gegenwart von Schwefelsäure zu Dinitrotoluol umgesetzt wird.

Die Selektivität der Toluolumsetzung wird im Wesentlichen durch die Schwefelsäurekonzentration in den beiden Reaktionsstufen des Verfahrens beeinflusst. Wie oben ausgeführt, führen in der Mononitrierung Schwefelsäurekonzentrationen < 70 Gew.-% H₂SO₄, bezogen auf das Gewicht der Schwefelsäure, zu einer verstärkten Oxidationsneigung der Salpetersäure, werden durch Oxidation von Toluol, MNT oder DNT Kresole, Phenole sowie deren Nitro- und Abbauprodukte erhalten. Umgekehrt führen zu hohe Schwefelsäurekonzentrationen auf der Dinitrierstufe durch die stets vorhandene Salpetersäure zu einer erhöhten Trinitrotoluolbildung.

Die angestrebte Vollständigkeit der Umsetzung in den Reaktionsstufen wird bei gegebener Verweilzeit ebenfalls durch die Schwefelsäurekonzentration sowie die gewählte Reaktionstemperatur beeinflusst. Sie ist zusätzlich von der Salpetersäurekonzentration in der schwefelsauren Phase abhängig. Sie ist ebenfalls von den in den Reaktionsstufen erzeugten Phasengrenzflächen abhängig, da in beiden Nitrierstufen das Reaktionsgemisch die Neigung hat, in eine organische, nur Spuren an Säuren enthaltende, und eine schwefelsaure Phase zu zerfallen.

Ein hoher spezifischer Einsatz an Salpetersäure bezogen auf die zu nitrierenden Komponenten fördert zwar deren Umsetzung, führt auf der anderen Seite jedoch zu deutlichen Salpetersäurebeladungen der Absäure, die in der Phasentrennung der Mononitrierstufe gewonnen wird, bzw. dem DNT, das in der Phasentrennung der Dinitrierstufe entnommen wird. Er führt mithin zu deutlichen Mengen an nicht umgesetzter Salpetersäure, die in nachgeschalteten Stufen aus beiden Massenströmen entfernt und aufbereitet werden müssen und dann wieder den Reaktionsstufen zugeführt werden können.

Es hat nicht an Versuchen gefehlt, die isotherme zweistufige Umsetzung von Toluol mit Salpetersäure in Gegenwart von Schwefelsäure hinsichtlich seiner Wirtschaftlichkeit zu verbessern, denn die Herstellung des Dinitrotoluols erfolgt industriell in einem so großen Maßstab, dass selbst geringe ökonomische Verbesserungen dieses wichtigen großtechnischen Verfahrens von großem wirtschaftlichen Interesse sind.

EP 903 336 A lehrt den Einsatz von bevorzugt 87 - 89 Gew.-%-iger Schwefelsäure, die durch Aufbereitung der Absäure aus der Mononitrierung hergestellt wird und mit deutlich geringerem Aufwand als Schwefelsäuren mit Gehalten > 89 Gew.-% gewinnbar ist. Der geringeren Schwefelsäurekonzentration wird dadurch Rechnung getragen, dass der Prozess statt in zwei, in drei Nitrierstufen durchgeführt wird. Dabei wird die in der Phasentrennung der zweiten Stufe gewonnene, das Dinitrotoluol enthaltende organische Phase einer dritten, als Polishing- Zone bezeichneten Reaktionsstufe zugeführt und dort mit einer Mischsäure, die einen aliquoten Anteil an Salpetersäure sowie die gesamte in das Verfahren frisch eingesetzte Schwefelsäure enthält, zur Reaktion gebracht. Grundlage des Verfahrens ist, dass die in die Polishing-Zone eingesetzte Schwefelsäure allein von dem Wassergehalt des aliquoten Salpetersäureeinsatzes sowie dem durch die in der Polishing-Zone ablaufenden Rest-Nitrierung gebildetem Reaktionswasser verdünnt wird. In der Polishing-Zone werden dabei Säurestärken erreicht, die sogar über den Säurestärken eines Standardverfahrens liegen. Nachteilig an dem Verfahren ist neben den zusätzlichen Investitionen und Betriebskosten der dritten Stufe, dass die Polishing-Zone nur einen Reaktor aufweist. Deshalb muss zur vollständigen MNT-Umsetzung soviel Salpetersäure zugesetzt werden, dass die anschließend gewonnene Absäure der dritten Stufe Salpetersäuregehalte von ca. 0,4 Gew.-% aufweist. Diese Salpetersäuregehalte bergen in Verbindung mit der erhöhten Schwefelsäurekonzentration zum einen die Gefahr einer verstärkten TNT-Bildung (US 3 157 706 A) und führen zum anderen erfahrungsgemäß zu Salpetersäuregehalten im abfließenden DNT von deutlich > 0,4 Gew.-% und somit zu deutlichen Mengen an nicht umgesetzter Salpetersäure.

Die Minimierung der Salpetersäureverluste über den DNT-Austrag ist Inhalt von EP 279 312 A2. EP 279 312 A2 lehrt ein Verfahren zur Abtrennung von Schwefelsäure und Salpetersäure aus den bei der Dinitrierung von Toluol mit Mischsäure erhaltenen Schwefelsäure und Salpetersäure enthaltenden Dinitrotoluolen, dadurch gekennzeichnet, dass man die nach der Abtrennung der Hauptmenge an Schwefelsäure und Salpetersäure erhaltenen Dinitrotoluole, die noch bis zu 6 Gew.-% Schwefelsäure und bis zu 5 Gew.-% Salpetersäure enthalten, mit bis zu 10 Gew.-% Wasser, bezogen auf die Menge an Dinitrotoluolen, vermischt und die sich danach abscheidende Schwefel- und Salpetersäure enthaltende wässrige Phase abtrennt. Dabei wird die DNT-Wäsche ein oder mehrstufig durchführt.

Eine gegenüber EP 279 312 A2 verbesserte Säurerückgewinnung lehrt EP 736 514 A1. Gegenstand von EP 736 514 A1 ist ein Verfahren zum Entfernen und Wiedergewinnen von Salpetersäure, Schwefelsäure und Stickoxiden aus den bei der Nitrierung von Toluol oder Mononitrotoluolen nach Abtrennung der Nitriersäure anfallenden rohen Dinitrotoluole, bei dem die rohen Dinitrotoluole mit einer verdünnten wässrigen Lösung von Salpetersäure, Schwefelsäure und salpetrigere Säure mehrstufig, insbesondere zwei bis vierstufig, im Gegenstrom extrahiert werden, dabei das Volumenverhältnis der Dinitrotoluole zu der wässrigen Lösung jeweils 1:3 bis 10:1, vorzugsweise 1: 1 bis 4:1, beträgt und der wässrige Extrakt direkt oder nach Aufkonzentrierung in die Nitrierung zurückgeführt wird.

In allen Extraktionsstufen wird zweckmäßig bei einer Temperatur über der Schmelztemperatur der Dinitrotoluole gearbeitet. Die Dichte der wässrigen Lösung sollte in allen Stufen anders, vorzugsweise geringer sein als die der Dinitrotoluole. Die verdünnte wässrige Lösung wird zweckmäßig innerhalb jeder Extraktionsstufe im Kreislauf geführt. Das gewünschte Volumenverhältnis der Dinitrotoluole zu der verdünnten wässrigen Lösung von Salpetersäure, Schwefelsäure und salpetriger Säure lässt sich über die Zugabe von Frischwasser in den Extraktionskreislauf der verdünnten Lösung der letzten Extraktionsstufe einstellen. Hierbei wird insbesondere das bei der Aufkonzentrierung des wässrigen Extraktes anfallende Kondensat zugesetzt. Die aus der ersten Extraktionsstufe abgezogene wässrige Lösung ist ein Salpetersäure-/Schwefelsäuregemisch mit 25 bis 40 Gew.-% Gesamtsäure. Diese wird allein oder vorzugsweise mit der bei der Mononitrotoluolendsäurebehandlung anfallenden Salpetersäure bis zu einem Gesamtsäuregehalt von 65 Gew.-%, berechnet als HNO₃, aufkonzentriert.

Im Hinblick auf die Minimierung nicht genutzter Salpetersäure ist der Salpetersäuregehalt der in der Phasentrennung der Mononitrierstufe anfallenden Absäure ebenfalls Gegenstand zahlreicher Untersuchungen.

US 2 947 791 A lehrt ein verbessertes kontinuierliches Verfahren zur Nitrierung von Toluol, dessen Verbesserung darin besteht, dass äquimolare Mengen an Salpetersäure (eine Komponente der Nitriersäure aus Salpeter- und Schwefelsäure, enthaltend 50 -60 Gew.-% Schwefelsäure, 20 - 40 Gew.-% Salpetersäure und 10 - 20 Gew.-% Wasser) und Toluol bei 50 bis 100°C derart in einem gut gerührten, aus zwei in Reihe geschalteten Reaktoren bestehenden System umgesetzt werden, dass in den ersten Reaktor 0,4 bis 0,7 mol Toluol pro mol Salpetersäure gespeist werden, das diesen Reaktor verlassende Reaktionsgemisch dann in dem zweiten Reaktor mit der restlichen Toluolmenge versetzt und umgesetzt wird.

Durch den durchgeführten Toluolsplit wird gemäß der Lehre von US 2 947 791 A auf der Mononitrierungsstufe ein Toluolumsatz > 95 % bei geringen Gehalten an Stickoxiden und Salpetersäure in der wässrigen Phase erhalten. In der folgenden Dinitrierung wird dann stets ein 5 - 10 % molarer Überschuss an Salpetersäure eingesetzt.

US 2 475 095 A lehrt, dass Restgehalte an Toluol in dem Reaktionsgemisch der Mononitrierung die folgende Phasentrennung des Reaktionsgemisches erschweren. US 2 475 095 A lehrt deshalb, bereits in die Mononitrierung gegenüber dem Toluol einen Salpetersäureüberschuss einzusetzen. Dabei sollte die Absäure der Mononitrierungsstufe einen Salpetersäuregehalt von 1 Gew.-% aufweisen.

Von einem Salpetersäureüberschuss in der Mononitrierung und damit einem deutlichen Gehalt an Salpetersäure in der Absäure der Mononitrierungsstufe, geht ebenfalls US 4 496 782 A aus. Zur Nutzung dieser Gehalte lehrt US 4 496 782 A, die Absäure der Mononitrierung gezielt mit weiterer Salpetersäure auf Gehalte > 2 Gew.-% aufzustocken, dann die aufgestockte Absäure mit einer aliquoten Mononitrotoluolmenge in einem gerührten Reaktor adiabat bei Temperaturen > 110°C so umzusetzen, dass in der nachfolgenden Phasentrennung Absäuren mit Salpetersäuregehalten < 0,25 Gew.-% gewonnen werden. Neben einem erhöhten technischen Aufwand birgt dieses Verfahren sicherheitstechnische Risiken.

DE 10 2004 005 913 A1 stellt den für die zweistufige isotherme Umsetzung von Toluol mit Salpetersäure in Gegenwart von Schwefelsäure notwendigen technischen Aufwand besonders heraus. Nach der Lehre von DE 10 2004 005 913 A1 ist es wesentlich, diesen gegenüber dem Stand der Technik (nach DE 10 2004 005 913 A1 sind das jeweils zwei- bis vierstufige Rührkesselkaskaden auf der Mononitrier- und Dinitrierstufe) zu reduzieren. DE 10 2004 005 913 A lehrt den Einsatz von einem Rührkessel auf der Mononitrierstufe und den Einsatz von zwei in Kaskade geschalteten Rührkesseln auf der Dinitrierstufe.

DE 10 2004 005 913 A1 lehrt weiterhin einen unvollständigen Toluolumsatz bei gleichzeitig signifikanten Salpetersäuregehalten in der Absäure (im erfindungsgemäßen Beispiel 0,96 Gew.-%) auf der Mononitrierstufe und den vollständigen Umsatz auf der Dinitrierstufe, der durch einen entsprechenden Salpetersäureüberschuss erreicht wird.

DE 10 2004 005 913 A1 begegnet dem Problem der deutlichen Mengen dem System entzogener, nicht umgesetzter Salpetersäure in der Absäure der Mononitrierung bzw. der Dinitrierung mit folgendem Hinweis: "Zur Minimierung der Verluste an Salpetersäure, die nicht in das Endprodukt umgewandelter Salpetersäure wird, wird, wie beispielsweise in EP 0 736 514 beschrieben, die Salpetersäure aus der Wäsche des Roh-DNT als schwache Säure mit einem Gesamtsäuregehalt von 23,73 bis 40 % Gesamtsäure zusammen mit der Salpetersäure aus der Abgaswäsche und der Strippung der Abfallsäure direkt oder nach Aufkonzentrierung in die Nitrierung zurückgeführt". DE 10 2004 005 913 ersetzt mithin den geringeren Aufwand in den Reaktionsstufen durch einen erhöhten Aufwand in den DNT- und Absäureaufarbeitungsstufen, beinhaltet zusätzlich die Gefahr unkontrolliert ablaufender weiterer Reaktionen auf der Mononitrierstufe sowie einer verstärkten Trinitrotoluol(TNT)-Bildung auf der Dinitrierstufe.

Es bleibt somit die Aufgabe, ein Verfahren zur Umsetzung von Toluol mit Salpetersäure zu Dinitrotoluol in Gegenwart von Schwefelsäure zur Verfügung zu stellen, das bei einem einfachen Anlagenaufbau einen hohen Toluolumsatz mit hoher Selektivität bezüglich DNT sowie eine hohe und selektive Nutzung der direkt in die Reaktionsstufen eingesetzten Salpetersäure gestattet.

Es ist bekannt, dass bei der angestrebten isothermen Nitrierung von Toluol bzw. Mononitrotoluol mit Salpetersäure in Gegenwart von Schwefelsäure die Nitrierung der Aromaten im Wesentlichen in der wässrigen der beiden Phasen erfolgt ( Ullmann's Enzyklopädie der technischen Chemie, 4.Auflage, Band 17, S.391, 1979, Verlag Chemie, Weinheim-New York ). Um miteinander zu reagieren, muss das Toluol in der Mononitrierung bzw. das Mononitrotoluol in der Dinitrierung durch die Grenzfläche zwischen den beiden Phasen diffundieren, um in der wässrigen Phase in Gegenwart von Schwefelsäure mit der dort vorhandenen Salpetersäure zu reagieren.

Es ist weiterhin bekannt, dass in solchen Fällen die effektive Geschwindigkeit der Reaktion in Abhängigkeit von den Reaktionsbedingungen wie Reaktionstemperatur, die Konzentration der zu reagierenden Komponenten in der organischen Phase sowie die Salpetersäurekonzentration und die Schwefelsäurekonzentration in der wässrigen Phase stark von der Größe der sich stets erneuernden Phasengrenzfläche abhängen kann und dass diese beispielsweise durch intensives Rühren vergrößert werden kann. Dies wirkt sich vorteilhaft auf die Reaktionsgeschwindigkeit aus.

Auch mögliche Abhängigkeiten der erzielbaren, sich stets erneuernden Phasengrenzfläche als Funktion der Art der Mischung und der Zusammensetzung eines aus zwei nicht mischbaren Flüssigkeiten bestehenden Systems sind in der Literatur beschrieben (J.M.Zaldivar et al., Chemical Engineering and Processing 34 (1995)), 529 ff. So wird unter anderem auch über entsprechende Abhängigkeiten bei der Mononitrierung von Toluol mit Salpetersäure in Gegenwart von Schwefelsäure berichtet (ibid).

Überraschenderweise wurde nun gefunden, dass die für die Umsetzung des Toluols beschriebenen grundsätzlichen Abhängigkeiten wesentlich ausgeprägter für die Umsetzung des Mononitrotoluols mit Salpetersäure in Gegenwart von Schwefelsäure zu Dinitrotoluol zu beobachten sind.

So ist auf beiden Reaktionsstufen der isothermen Nitrierung (Mononitrierung und Dinitrierung) von Toluol zu Dinitrotoluol mit Salpetersäure in Gegenwart von Schwefelsäure die erzielbare Größe der sich stets erneuernde Phasengrenzfläche nicht nur von der in die Reaktionssysteme eingetragenen Mischenergie abhängig. Sie ist ebenfalls abhängig von dem Gewichts-Verhältnis der in den Reaktionssystemen vorhandenen Phasen. Sie ist ebenfalls davon abhängig, welche Phase in welcher Phase dispergiert ist.

So werden bei Gewichts-Verhältnissen der wässrigen zur organischen Phase von > 2 : 1, bevorzugt > 3 : 1, besonders bevorzugt > 3,5 : 1 auf der Mononitrierstufe und > 1,5 : 1, bevorzugt > 3 : 1, besonders bevorzugt > 3,5 : 1 auf der Dinitrierstufe und gleichzeitigem Vorliegen einer Dispersion der organischen Phase in der wässrigen Phase (und nicht eine inverse Dispersion bzw. eine "Tröpfchen in Tröpfchen"-Emulsion) so große, sich stets erneuernde Phasengrenzflächen zugänglich, dass überraschenderweise trotz des Einsatzes von < 2,06 mol Salpetersäure pro mol Toluol in das Gesamtverfahren ein weitgehend vollständiger Umsatz des Toluols zu Mononitrotoluol sowie des Mononitrotoluols zu Dinitrotoluol erzielt wird.

Die Erfindung betrifft ein Verfahren zur Herstellung von Dinitrotoluol durch Nitrierung von Toluol mit Nitriersäure, bei dem
a) Toluol mit Nitriersäure zu Mononitrotoluol umgesetzt wird, wobei ein Mononitrotoluol enthaltendes Reaktionsgemisch erhalten wird, und
b) das Mononitrotoluol enthaltende Reaktionsgemisch in eine Mononitrotoluol enthaltende organische Phase und eine Schwefelsäure enthaltende wässrige Phase aufgetrennt wird, und
c) die Mononitrotoluol enthaltende organische Phase mit Nitriersäure umgesetzt wird, wobei ein Dinitrotoluol enthaltendes Reaktionsgemisch erhalten wird, und
d) das Dinitrotoluol enthaltende Reaktionsgemisch in eine Dinitrotoluol enthaltende organische Phase und eine Schwefelsäure enthaltende wässrige Phase aufgetrennt wird,
   dadurch gekennzeichnet, dass
e) das Gewichtsverhältnis von wässriger zur organischer Phase in der Nitrierung in dem Schritt a) > 2 : 1, bevorzugt > 3 : 1, ganz besonders bevorzugt > 3,5 : 1 und in dem Schritt c) > 1,5 : 1, bevorzugt > 3 :1, ganz besonders bevorzugt > 3,5 : 1 beträgt, und
f) in den Schritten a) und c) jeweils die organische Phase in der wässrigen Phase dispergiert wird, und
g) insgesamt pro mol Toluol weniger als 2,06 mol an Salpetersäure eingesetzt wird.

Wesentlich an dem erfindungsgemäßen Verfahren ist, dass in der Mononitrierstufe (Schritt a) das Gewichts-Verhältnis der wässrigen zur organischen Phase auf Werte > 2 : 1, bevorzugt > 3 : 1, besonders bevorzugt > 3,5 : 1, und in der Dinitrierstufe (Schritt c ) auf Werte > 1,5 : 1, bevorzugt > 3 : 1, besonders bevorzugt > 3,5 : 1 eingestellt wird und in beiden Schritten jeweils die organische Phase als disperse Phase in der wässrigen Phase (homogene Phase) dispergiert wird und gleichzeitig insgesamt weniger als 2,06 mol Salpetersäure pro mol Toluol in das Verfahren (Schritte a)- d)) eingespeist werden.

Von besonderer Bedeutung bei dem erfindungsgemäßen Verfahren ist, dass das Reaktionsgemisch in beiden Reaktionsschritten a) und c) in Form einer Dispersion vorliegt, in der jeweils die organische in der wässrigen als homogener Phase dispergiert ist. Dabei wird bevorzugt der Eintrag an Misch- bzw. Dispergierenergie so gewählt, dass einerseits die gewünschten Dispersionen mit sehr großen Phasengrenzflächen erzeugt werden und andererseits eine Bildung stabiler "Tropfen in Tropfen" - Emulsionen vermieden wird. Die in die Reaktionsschritten vorteilhaft einzubringende Misch- bzw. Dispergierenergie kann in einfachen Versuchen leicht ermittelt werden. Auch bei Einhaltung der erfindungsgemäßen Phasenverhältnisse ist sie abhängig von der gewählten Reaktor-und Rührergeometrie sowie den physikalischen Daten der Reaktionsgemische. Einmal eingestellt, kann der Zustand der in den Reaktionssystemen vorliegenden Dispersionen jedoch vorteilhaft über die Leitfähigkeit der homogen durchmischten Reaktionsmischungen kontrolliert werden.

Bevorzugt wird dabei die Umsetzung in Schritt a) und / oder in Schritt c) isotherm durchgeführt.

In einer bevorzugten Ausführungsform erfolgt die Umsetzung in den Reaktionsschritten a) und c) jeweils in Kaskaden von Reaktoren, in denen eine Vermischung stattfindet, besonders bevorzugt in Kaskaden mit jeweils 2 bis ≤ 4 Reaktoren. In einer besonders bevorzugten Form werden als Reaktoren Schleifenreaktoren eingesetzt, die Umwälzpumpen und Wärmeaustauscher umfassen.

In einer ganz besonders bevorzugten Form werden in der Mononitrierstufe zwei in Reihe geschaltete Schleifenreaktoren, die Umwälzpumpen und Wärmeaustauscher enthalten, eingesetzt. In der Dinitrierstufe werden dann zwei in Reihe geschaltete Schleifenreaktoren, die Umwälzpumpe und Wärmeaustauscher umfassen, sowie zusätzlich ein weiterer Schleifenreaktor, der eine Umwälzpumpe, aber keinen Wärmeaustauscher umfasst, eingesetzt. Dabei erfolgt die Dimensionierung der Umwälzpumpen so, dass stets eine Dispersion der organischen in der homogenen wässrigen Phase erfolgt. Bevorzugt erfolgt in dieser Ausführungsform in wenigstens einem der eingesetzten Reaktoren zusätzlich eine Leitfähigkeitsmessung zur kontinuierlichen Kontrolle des Zustands der umgewälzten Dispersion.

Den Reaktionsschritten a) und c) ist jeweils ein Phasentrennschritt b) und d) nachgeschaltet. Dabei können alle zur Phasentrennung geeignete Apparate zum Einsatz kommen. Es sind sowohl dynamisch wie auch statische Abscheider geeignet. In einer bevorzugten Ausführungsform kommen auf beiden Stufen (Schritte b) und d)) statische Abscheider zum Einsatz.

In dem erfindungsgemäßen Verfahren wird in Schritt a) das Toluol der Mononitrierstufe zugeführt. Bevorzugt erfolgt die Zufuhr des Toluols in den ersten Reaktor, ein Split der Toluolzugabe auf mehrere Reaktoren ist aber ebenfalls möglich. Bevorzugt wird das in den ersten Reaktor eingespeiste Toluol über eine oder mehrere Düsen mit der Nitriersäure vermischt dem Reaktor zugegeben. Dabei wird bevorzugt vorab die Schwefelsäure enthaltende wässrige Phase aus Schritt d) (Absäure der Dinitrierstufe) mit Salpetersäure vermischt und so die Nitriersäure hergestellt. Es kann aber auch frische Schwefelsäure oder ein Gemisch aus frischer Schwefelsäure und der Absäure aus der Dinitrierstufe eingesetzt werden.

Eine getrennte Zugabe des Toluols sowie der Nitriersäure sind ebenfalls möglich. Die getrennte Zugabe der Salpetersäure sowie der Schwefelsäure, z.B. der Absäure aus der Dinitrierstufe, sind ebenfalls möglich. Das Gewichts-Verhältnis der Phasen in den Reaktoren der Mononitrierstufe wird bevorzugt über die Menge der in die Reaktoren eingespeiste Schwefelsäure, z.B. der Absäure der Dinitrierstufe, eingestellt. Aber auch eine Rückführung der in Schritt b) erhaltenen Schwefelsäure enthaltenden wässrigen Phase aus der Mononitrierstufe ist möglich, wobei die rückgeführte Schwefelsäure enthaltende wässrige Phase bevorzugt auf den ersten Reaktor der Stufe gespeist wird. Eine Zugabe auf mehrere Reaktoren der Stufe ist jedoch ebenfalls möglich. Auch ein Split der der Mononitrierung zugeführten Salpetersäure auf mehrere Reaktoren ist möglich.

In einer besonders bevorzugten Variante werden in die Mononitrierung (Schritt a)) pro mol Toluol ≤ 1,03 mol Salpetersäure eingesetzt. Insgesamt werden in dem Verfahren über beide Nitrierstufen < 2,06 mol Salpetersäure pro mol Toluol eingesetzt.

Die Umsetzung des Toluols erfolgt auf der Mononitrierstufe (Schritt a)) in einem Temperaturbereich von 30 bis 70°C, wobei die Reaktoren der Mononitrierstufe bei gleicher Reaktionstemperatur betrieben werden können. Dem Reaktionsfortschritt angepasste, unterschiedliche Reaktionstemperaturen in den einzelnen Reaktoren sind jedoch ebenfalls möglich.

Bevorzugt werden die in den Reaktoren der Mononitrierstufe (Schritt a)) vorliegenden Dispersiönen mit Hilfe von Leitfähigkeitsmessungen kontinuierlich überwacht. So werden unzulässige Abweichungen, die beispielsweise erkennbar sind an einem signifikanten Abfall der Leitfähigkeit der umgewälzten Reaktionsgemische, durch Änderungen der über die Misch- bzw. Dispergiereinrichtungen in die Reaktoren eingetragene Mischenergie oder aber bevorzugt über eine Änderung des Phasenverhältnisses in den Reaktoren der Nitrierstufe korrigiert.

Bei dem erfindungsgemäßen Verfahren wird ein sehr hoher Toluolumsatz mit einer sehr effektiven Ausnutzung der in die Mononitrierstufe eingespeisten Salpetersäure erreicht. Die in der nachfolgende Phasentrennung gewonnene Schwefelsäure enthaltende wässrige Phase (Absäure) weist typischerweise Salpetersäuregehalte von < 0,1 Gew.-%, bezogen auf das Gewicht der Absäure, auf. Diese niedrigen Gehalte führen zu dem sehr niedrigen spezifischen Salpetersäurebedarf des erfindungsgemäßen Verfahrens. Zusätzlich verringern sie den zur Aufkonzentrierung der Absäure der Mononitrierstufe notwendigen Aufwand.

In dem erfindungsgemäßen Verfahren wird die in der Phasentrennung der Mononitrierstufe (Schritt b)) erhaltene organische Phase bevorzugt ohne weitere Aufarbeitung den Reaktoren der Dinitrierstufe (Schritt c)) zugeführt. Bevorzugt erfolgt die Zufuhr der MNT enthaltenden organischen Phase in den ersten Reaktor, ein Split auf mehrere Reaktoren ist aber ebenfalls möglich. Bevorzugt wird das in den ersten Reaktor eingespeiste MNT über eine oder mehrere Düsen mit der Nitriersäure vermischt dem Reaktor zugegeben. Dabei wird bevorzugt vorab die Schwefelsäure enthaltende wässrige Phase aus Schritt b) (Absäure der Mononitrierstufe) ggf. aufbereitet und mit Salpetersäure vermischt und so die Nitriersäure hergestellt. Es kann aber auch frische Schwefelsäure oder ein Gemisch aus frischer Schwefelsäure und der Absäure aus der Mononitrierstufe eingesetzt werden. Eine getrennte Zugabe der MNT enthaltenden organischen Phase aus Schritt b) sowie der Nitriersäure sind ebenfalls möglich. Die getrennte Zugabe der Salpetersäure sowie der Schwefelsäure, z.B. der aufbereiteten Absäure aus der Mononitrierstufe, sind ebenfalls möglich. Das Gewichts-Verhältnis der Phasen in den Reaktoren der Dinitrierstufe (Schritt c)) wird bevorzugt über die Menge der in die Reaktoren eingespeiste Schwefelsäure, z.B. der aufbereiteten Absäure aus der Mononitrierstufe, eingestellt. Aber auch eine Rückführung der in Schritt d) erhaltenen Schwefelsäure enthaltenden wässrigen Phase aus der Dinitrierstufe ist möglich, wobei die rückgeführte Schwefelsäure enthaltende wässrige Phase bevorzugt auf den ersten Reaktor der Stufe gespeist wird. Eine Zugabe auf mehrere Reaktoren der Stufe ist jedoch ebenfalls möglich. Auch ein Split der der Dinitrierung zugeführten Salpetersäure auf mehrere Reaktoren ist möglich.

In einer besonders bevorzugten Variante werden in die Dinitrierung (Schritt c)) pro mol Mononitrotoluol ≤ 1,03 mol Salpetersäure eingesetzt. Insgesamt werden in dem Verfahren über beide Nitrierstufen < 2,06 mol Salpetersäure pro mol Toluol eingesetzt.

Die Umsetzung des Mononitrotoluols erfolgt auf der Dinitrierstufe (Schritt c)) in einem Temperaturbereich von 55 bis 80°C, wobei die Reaktoren der Dinitrierstufe bei gleicher Reaktionstemperatur betrieben werden können. Dem Reaktionsfortschritt angepasste, unterschiedliche Reaktionstemperaturen in den einzelnen Reaktoren sind jedoch ebenfalls möglich.

Bevorzugt werden die in den Reaktoren der Dinitrierstufe (Schritt c)) vorliegenden Dispersionen mit Hilfe von Leitfähigkeitsmessungen kontinuierlich überwacht. So werden unzulässige Abweichungen, die beispielsweise erkennbar sind an einem signifikanten Abfall der Leitfähigkeit der umgewälzten Reaktionsgemische, durch Änderungen der über die Misch- bzw. Dispergiereinrichtungen in die Reaktoren eingetragene Mischenergie oder aber bevorzugt über eine Änderung des Phasenverhältnisses in den Reaktoren der Nitrierstufe korrigiert.

Bei dem erfindungsgemäßen Verfahren wird ein sehr hoher Mononitrotoluolumsatz mit einer sehr effektiven Ausnutzung der in die Dinitrierstufe eingespeisten Salpetersäure erreicht. Die in der nachfolgenden Phasentrennung gewonnene Schwefelsäure enthaltende wässrige Phase (Absäure) weist typischerweise Salpetersäuregehalte von < 0,2 Gew.-%, bezogen auf das Gewicht der Absäure, auf. Die Beladungen des in Schritt d) erhaltenen DNT enthaltenden organischen Phase mit Salpetersäure liegen dabei bei < 0,4 Gew.-% HNO₃, bezogen auf das Gewicht der organischen Phase.

Insgesamt führt das erfindungsgemäße Verfahren zu einer DNT-Ausbeute bezogen auf die eingesetzte Salpetersäure von > 97,7 %. So hohe DNT-Ausbeuten bezogen auf die eingesetzte Salpetersäure sind mit den Verfahren nach dem Stand der Technik nur durch aufwendige Aufbereitungen der in der Mononitrierung gewonnenen Absäure bzw. in nachgeschalteten Extraktionen und/oder Wäschen gewonnenen wässriger Phasen erzielbar.

Somit zeichnet sich das erfindungsgemäße Verfahren gegenüber den Verfahren nach dem Stand der Technik durch einen sehr hohe DNT-Ausbeute bezogen auf die eingesetzte Salpetersäure bei gleichzeitig einfachem Anlagenaufbau aus. Der geringe Salpetersäurebedarf wird dabei ohne aufwendige und energieintensive zusätzliche Salpetersäurerückgewinnungen erzielt, die ansonsten gemäß dem Stand der Technik zur Erzielung geringer spezifischer Salpetersäureverbräuche angewandt werden.

Wesentlich für die geringen spezifischen Salpetersäureverbräuche ohne Aufbereitung etwaiger Absäuren und Waschwässer ist, dass in beiden Reaktionsstufen die organische in der wässrigen Phase dispergiert wird. Dies wird durch die Vorgabe bestimmter Gewichts-Verhältnisse der Phasen in den Reaktionsschritten a) und c) ermöglicht bzw. stark erleichtert. Dadurch werden bei geeignet gewählten Reaktorkonfigurationen gegenüber einer invertierten Dispersion bei gleichem Energieeintrag stark vergrößerte, sich schnell erneuernde Phasengrenzflächen zwischen organischer und wässriger Phase zugänglich. Dadurch werden die überraschend hohen DNT-Ausbeuten bezogen auf die eingesetzte Salpetersäure bei gegenüber dem Stand der Technik etwa gleicher Schwefelsäurekonzentration und geringeren Salpetersäurekonzentrationen in den Reaktionsstufen möglich.

Von besonderer Bedeutung sind dabei die Reaktionsverhältnisse in der Dinitrierstufe (Schritt c)). Durch die im erfindungsgemäßen Verfahren erzielbaren großen, sich schnell erneuernden Phasengrenzflächen wird die Transportlimitierung der Umsetzung des Mononitrotoluols weitgehend aufgehoben. Somit sind zur vollständigen MNT-Umsetzung bei etwa gleicher Schwefelsäurekonzentration geringere Restkonzentrationen an Salpetersäure in dem die Stufe verlassenden Reaktionsgemisch erforderlich. Die geringeren Gehalte an Salpetersäure führen ebenfalls zu deutlich geringeren Salpetersäurebeladungen der in der anschließenden Phasentrennung (Schritt d)) gewonnenen Dinitrotoluol enthaltenden organischen Phase und somit zu einer deutlichen Absenkung der dem Reaktionssystem a) - d) entzogenen, nicht umgesetzten Salpetersäure.

### Beispiele:

### Allgemeines

Die Nitrierungen werden in einer kontinuierlich betreibbaren 2-stufigen Laborapparatur, jeweils bestehend aus
- Einsatzvorlagen mit Dosierpumpen,
- zwei in Reihe geschalteten Reaktoren (Rührkessel mit Heiz-/Kühl-Mänteln und schnell laufenden Laborrührern),
- einen in den Ablauf des zweiten Reaktors installierten Abscheider sowie
- nachgeschalteten Vorlagen zum Auffangen der separierten Phasen
durchgeführt, wobei jeweils über eine zusätzliche Dosierpumpe die aufgefangene wässrige Phase auf den ersten Reaktor der jeweiligen Kaskade zurückgeführt werden kann.

Bei der Durchführung der Nitrierungen wird jeweils Schwefelsäure in den im kontinuierlichen Betrieb zu erwartenden Konzentrationen in der inertisierten Apparatur vorgelegt, werden dann die Komponenten in den unten angegebenen Gewichtsverhältnissen und Massen dosiert und dabei der Mischenergieeintrag in die Reaktoren über die Rührer im Hinblick auf einen optimalen Wert der in den fein dispergierten Reaktionssystemen gemessenen Leitfähigkeiten eingestellt.

Nach Erreichen der des stationären Gleichgewichts werden die aus den Abscheidern ablaufenden organischen Phasen hinsichtlich ihres Umsatzes, im Fall der DNT-Phase zusätzlich hinsichtlich ihrer Hauptkomponentenzusammensetzung und ihrer Salpetersäurebeladung, analysiert. Es werden auch die aus den Abscheidern ablaufenden wässrigen Phasen hinsichtlich ihrer Salpetersäuregehalte untersucht.

Die Phasen werden zusätzlich qualitativ auf Nebenprodukte analysiert. Dabei werden in geringem Maß Nitrokresole und Nitrobenzoesäuren in unterschiedlichen Nitriergraden gefunden.

### Beispiel 1 ( nicht erfindungsgemäß)

Es werden kontinuierlich pro Stunde 720 g einer 96%igen Schwefelsäure mit 331 g einer 98,5 %igen Salpetersäure (zu Nitriersäure) vermischt, diese Mischung kontinuierlich mit 685,7 g/h rohem MNT aus der MNT-Stufe in den ersten Reaktor der DNT-Stufe dosiert, dort bei einer Temperatur von 70°C und Leitfähigkeiten von ca. 90 mS in der Reaktorenkaskade umgesetzt. Das ausreagierte Reaktionsgemisch wird in dem nachfolgenden Abscheider in eine organische und eine wässrige Phase getrennt.

Die wässrige Phase der Dinitrierstufe wird wiederum kontinuierlich pro Stunde mit 331 g einer 98,5 %igen Salpetersäure vermischt, diese Mischung kontinuierlich mit 460,7 g /h Toluol in den ersten Reaktor der MNT-Stufe dosiert, dort bei einer Temperatur von 50°C und Leitfähigkeiten von ca. 80 mS in der Reaktorenkaskade umgesetzt. Das in den Abscheider überlaufende Reaktionsgemisch wird dort in eine organische und eine wässrige Phase getrennt, dann die organische Phase kontinuierlich der Dinitrierstufe zugeführt, die wässrige an eine Aufarbeitung abgegeben.

Die erzielten Ergebnisse sind in Tabelle 1 zusammengestellt.

### Beispiel 2 (erfindungsgemäß)

Es werden kontinuierlich pro Stunde 715 g einer 96%igen Schwefelsäure mit 328 g einer 98,5 %igen Salpetersäure (zu Nitriersäure) vermischt, diese Mischung kontinuierlich mit 685,7 g/h rohem MNT aus der MNT-Stufe sowie 2190 g/h wässriger Phase aus dem Abscheider der DNT-Stufe in den ersten Reaktor der DNT-Stufe dosiert, dort bei einer Temperatur von 70°C und Leitfähigkeiten von ca. 160 mS in der Reaktorenkaskade umgesetzt. Das ausreagierte Reaktionsgemisch wird in dem nachfolgenden Abscheider in eine organische und eine wässrige Phase getrennt.

Die wässrige Phase der Dinitrierstufe wird wiederum kontinuierlich pro Stunde mit 328 g einer 98,5 %igen Salpetersäure vermischt, diese Mischung kontinuierlich mit 460,7 g /h Toluol sowie 2310 g/h wässriger Phase aus dem Abscheider der MNT-Stufe in den ersten Reaktor der MNT-Stufe dosiert, dort bei einer Temperatur von 50°C und Leitfähigkeiten von ca. 120 mS in der Reaktorenkaskade umgesetzt. Das in den Abscheider überlaufende Reaktionsgemisch wird dort in eine organische und eine wässrige Phase getrennt, dann die organische Phase kontinuierlich der Dinitrierstufe zugeführt, die wässrige an eine Aufarbeitung abgegeben.

Die erzielten Ergebnisse sind ebenfalls in Tabelle 1 zusammengestellt.

### Beispiel 3 (erfindungsgemäß)

Es werden kontinuierlich pro Stunde 275,4 g einer 92%igen Schwefelsäure mit 474,9 g einer 68 %igen Salpetersäure (zu Nitriersäure) vermischt, diese Mischung kontinuierlich mit 685,7 g/h rohem MNT aus der MNT-Stufe in den ersten Reaktor der DNT-Stufe dosiert, dort bei einer Temperatur von 70°C und Leitfähigkeiten von ca.160 mS in der Reaktorenkaskade umgesetzt. Das ausreagierte Reaktionsgemisch wird in dem nachfolgenden Abscheider in eine organische und eine wässrige Phase getrennt.

Die wässrige Phase der Dinitrierstufe wird wiederum kontinuierlich pro Stunde mit 474,9 g einer 68 %igen Salpetersäure vermischt, diese Mischung kontinuierlich mit 460,7 g /h Toluol in den ersten Reaktor der MNT-Stufe dosiert, dort bei einer Temperatur von 50°C und Leitfähigkeiten von ca. 120 mS in der Reaktorenkaskade umgesetzt. Das in den Abscheider überlaufende Reaktionsgemisch wird dort in eine organische und eine wässrige Phase getrennt, dann die organische Phase kontinuierlich der Dinitrierstufe zugeführt, die wässrige Phase an eine Aufarbeitung abgegeben.

Die erzielten Ergebnisse sind ebenfalls in Tabelle 1 zusammengestellt.

Aus der Tabelle 1 in der Spalte Salpetersäure Distufe" ist zu erkennen, dass die notwendige Menge an Salpetersäure in den erfindungsgemäßen Beispielen 2 und 3 (bei dem das Gewichtsverhältnis von wässriger zur organischer Phase in der Nitrierung in dem Schritt a) > 2 :1 und in dem Schritt c) > 1,5 : 1 beträgt) niedriger ist als in dem nicht erfindungsgemäßen Beispiel 1. Das resultiert daraus, dass unter den optimierten Fahrbedingungen des erfindungsgemäßen Verfahrens die Salpetersäure nahezu vollständig genutzt und damit verbraucht wird, was an der ausgeschleusten Menge an Salpetersäure in der Spalte "HNO₃ Rest in Absäure Di" in Tabelle zu erkennen ist.

Analoge Aussagen gelten ebenfalls für die Mononitrierstufe, wie die Angaben der Spalten "Salpetersäure Monostufe" und "HNO₃-Rest in Abs.-Mono" zeigen.

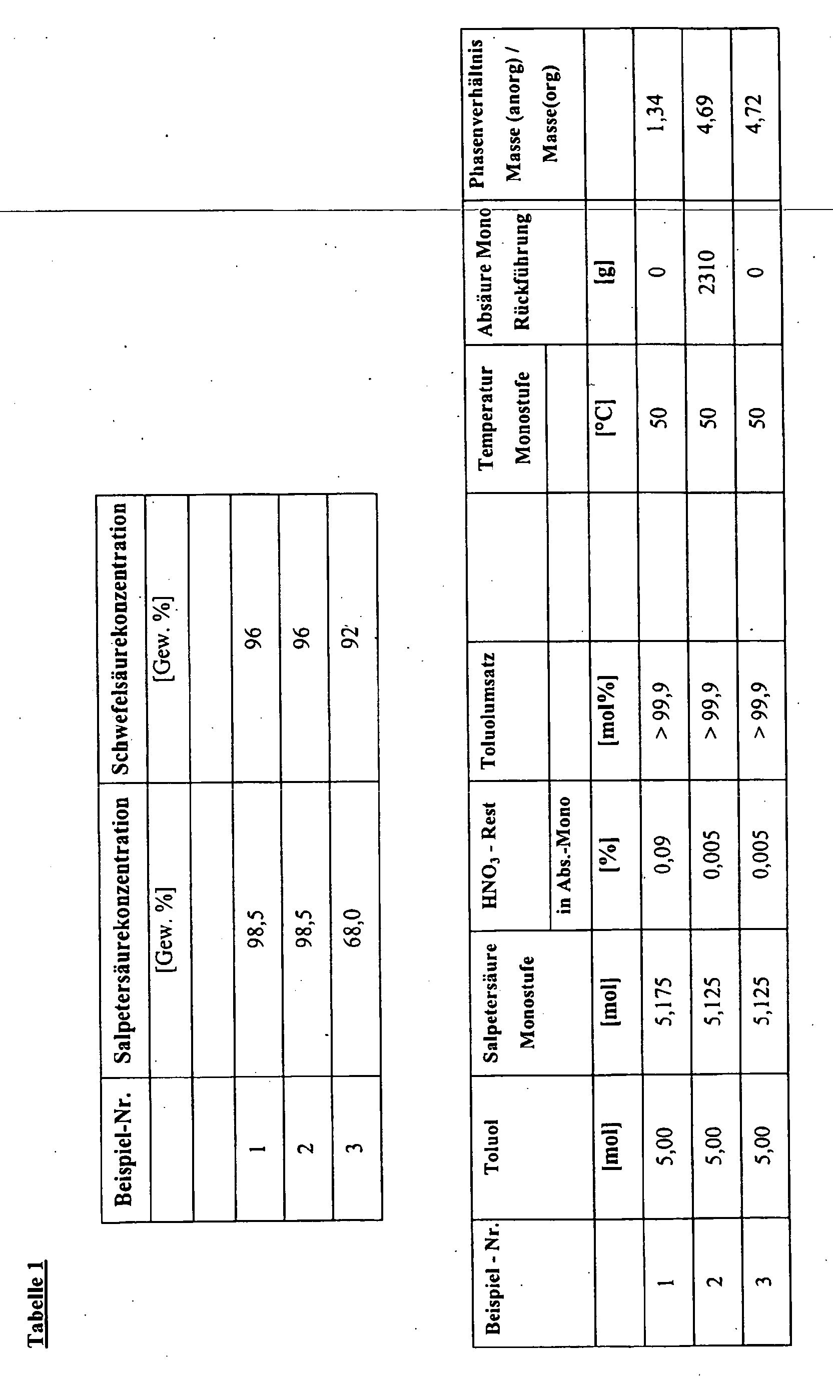

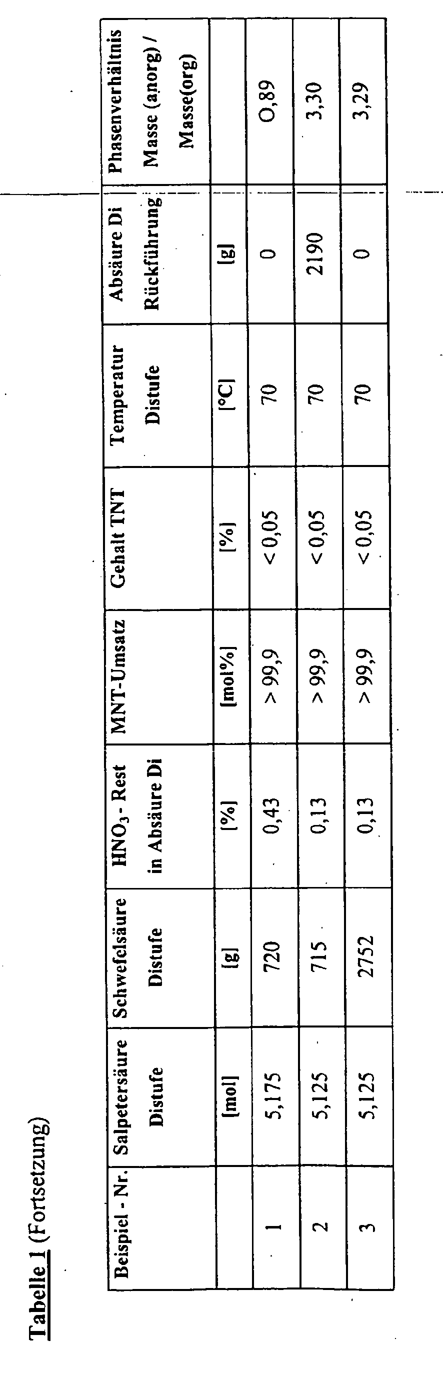

## Patentansprüche

1. Verfahren zur Herstellung von Dinitrotoluol durch Nitrierung von Toluol mit Nitriersäure, bei dem
a) Toluol mit Nitriersäure zu Mononitrotoluol umgesetzt wird, wobei ein Mononitrotoluol enthaltendes Reaktionsgemisch erhalten wird, und
b) das Mononitrotoluol enthaltende Reaktionsgemisch in eine Mononitrotoluol enthaltende organische Phase und eine Schwefelsäure enthaltende wässrige Phase aufgetrennt wird, und
c) die Mononitrotoluol enthaltende organische Phase mit Nitriersäure umgesetzt wird, wobei ein Dinitrotoluol enthaltendes Reaktionsgemisch erhalten wird, und
d) das Dinitrotoluol enthaltende Reaktionsgemisch in eine Dinitrotoluol enthaltende organische Phase und eine Schwefelsäure enthaltende wässrige Phase aufgetrennt wird;
**dadurch gekennzeichnet, dass**
e) das Gewichtsverhältnis von wässriger zur organischer Phase in der Nitrierung in dem Schritt a) > 2 :1 und in dem Schritt c) > 1,5 : 1 beträgt, und
f) in den Schritten a) und c) jeweils die organische Phase in der wässrigen Phase dispergiert wird, und
g) insgesamt pro mol Toluol weniger als 2,06 mol an Salpetersäure eingesetzt wird.

2. Verfahren nach Anspruch 1, bei dem Teile der in Schritt d) erhaltenen Schwefelsäure enthaltenden wässrigen Phase in die Umsetzung in Schritt c) zurückgeführt werden, so dass in der Phasentrennung in Schritt d) ein Gewichts-Verhältnis der Schwefelsäure enthaltenden wässrige Phase zu der Dinitrotoluol enthaltenden organischen Phase von > 1,5 eingestellt wird.

3. Verfahren nach Anspruch 2, bei dem die Teile der in Schritt d) erhaltenen Schwefelsäure enthaltenden wässrigen Phase auf den ersten Reaktor einer Kaskade aus ≤ 4 Reaktoren zurückgeführt werden.

4. Verfahren nach Anspruch 1, bei dem die in Schritt d) erhaltene Schwefelsäure enthaltende wässrige Phase vollständig oder teilweise in die Umsetzung in Schritt a) zurückgeführt wird.

5. Verfahren nach Anspruch 1, bei dem die in Schritt b) erhaltene Schwefelsäure enthaltende wässrige Phase vollständig oder teilweise in die Umsetzung in Schritt c) eingesetzt wird.

6. Verfahren nach Anspruch 1, bei dem die Umsetzung in Schritt a) und / oder Schritt c) isotherm durchgeführt wird.

7. Verfahren nach Anspruch 1, bei dem für die Phasentrennung in Schritt b) und / oder Schritt d) statische Abscheider eingesetzt werden.

8. Verfahren nach Anspruch 1, bei dem die Umsetzung in Schritt a) und / oder in Schritt c) in einer Kaskade aus ≤ 4 Reaktoren durchgeführt wird.

9. Verfahren nach Anspruch 8, bei dem der letzte Reaktor in Schritt c) ein Rohrreaktor ist.

10. Verfahren nach Anspruch 1, bei dem die Umsetzung in Schritt a) und / oder in Schritt c) in Schleifenreaktoren erfolgt.

11. Verfahren nach Anspruch 1, bei dem in Schritt a) pro Mol Toluol ≤ 1,03 Mol Salpetersäure eingesetzt werden.

12. Verfahren nach Anspruch 1, bei dem in Schritt c) pro Mol Mononitrotoluol ≤ 1,03 Mol Salpetersäure eingesetzt werden.

## Claims

1. A process for the production of dinitrotoluene by the nitration of toluene with nitrating acid, in which
a) toluene is reacted with nitrating acid to form mononitrotoluene, a reaction mixture being obtained which contains mononitrotoluene, and
b) the reaction mixture containing mononitrotoluene is separated into an organic phase containing mononitrotoluene and an aqueous phase containing sulfuric acid, and
c) the organic phase containing mononitrotoluene is reacted with nitrating acid, a reaction mixture being obtained which contains dinitrotoluene, and
d) the reaction mixture containing dinitrotoluene is separated into an organic phase containing dinitrotoluene and an aqueous phase containing sulfuric acid,
**characterised in that**
e) the weight ratio of aqueous to organic phase in the nitration in step a) is > 2 : 1 and in step c) > 1.5 : 1, and
f) in steps a) and c) the organic phase is dispersed in the aqueous phase in each case, and
g) overall, less than 2.06 moles of nitric acid are used per mole of toluene.

2. The process according to claim 1, in which parts of the aqueous phase containing sulfuric acid obtained in step d) are recycled into the reaction in step c) so that, in the phase separation in step d), a weight ratio of the aqueous phase containing sulfuric acid to the organic phase containing dinitrotoluene of > 1.5 is established.

3. The process according to claim 2, in which the parts of the aqueous phase containing sulfuric acid obtained in step d) are recycled to the first reactor of a cascade of ≤ 4 reactors.

4. The process according to claim 1, in which the aqueous phase containing sulfuric acid obtained in step d) is recycled completely or partly into the reaction in step a).

5. The process according to claim 1, in which the aqueous phase containing sulfuric acid obtained in step b) is fed completely or partly into the reaction in step c).

6. The process according to claim 1, in which the reaction in step a) and/or step c) is carried out isothermally.

7. The process according to claim 1, in which static separators are used for the phase separation in step b) and/or step d).

8. The process according to claim 1, in which the reaction in step a) and/or in step c) is carried out in a cascade of ≤ 4 reactors.

9. The process according to claim 8, in which the last reactor in step c) is a tubular reactor.

10. The process according to claim 1, in which the reaction in step a) and/or in step c) takes place in loop reactors.

11. The process according to claim 1, in which ≤ 1.03 moles of nitric acid are used per mole of toluene in step a).

12. The process according to claim 1, in which ≤ 1.03 moles of nitric acid are used per mole of mononitrotoluene in step c).

## Revendications

1. Procédé pour la préparation de dinitrotoluène par nitration de toluène avec un mélange sulfonitrique, dans lequel
a) on fait réagir du toluène avec un mélange sulfonitrique en mononitrotoluène, un mélange réactionnel contenant du mononitrotoluène étant obtenu, et
b) on sépare le mélange réactionnel contenant du mononitrotoluène en une phase organique contenant du mononitrotoluène et une phase aqueuse contenant de l'acide sulfurique, et
c) on fait réagir la phase organique contenant du mononitrotoluène avec un mélange sulfonitrique, un mélange réactionnel contenant du dinitrotoluène étant obtenu, et
d) on sépare le mélange réactionnel contenant du dinitrotoluène en une phase organique contenant du dinitrotoluène et une phase aqueuse contenant de l'acide sulfurique,
**caractérisé en ce que**
e) le rapport massique de la phase aqueuse à organique dans la nitration dans l'étape a) > 2 : 1 et dans l'étape c) > 1,5 : 1, et
f) on disperse dans les étapes a) et c) à chaque fois la phase organique dans la phase aqueuse, et
g) on utilise au total par mole de toluène moins de 2,06 mole d'acide nitrique.

2. Procédé selon la revendication 1, dans lequel des parties de la phase aqueuse contenant de l'acide sulfurique obtenue dans l'étape d) sont renvoyées dans la réaction dans l'étape c), de telle sorte qu'un rapport massique de la phase aqueuse contenant de l'acide sulfurique à la phase organique contenant du dinitrotoluène est dans la séparation de phase de l'étape d) ajusté à > 1,5.

3. Procédé selon la revendication 2, dans lequel les parties de la phase aqueuse contenant de l'acide sulfurique obtenue dans l'étape d) sont renvoyées dans le premier réacteur d'une cascade de ≤ 4 réacteurs.

4. Procédé selon la revendication 1, dans lequel la phase aqueuse contenant de l'acide sulfurique obtenue dans l'étape d) est totalement ou partiellement renvoyée dans la réaction de l'étape a).

5. Procédé selon la revendication 1, dans lequel la phase aqueuse contenant de l'acide sulfurique obtenue dans l'étape b) est totalement ou partiellement utilisée dans la réaction de l'étape c).

6. Procédé selon la revendication 1, dans lequel la réaction dans l'étape a) et/ou l'étape c) est réalisée de manière isotherme.

7. Procédé selon la revendication 1, dans lequel on utilise pour la séparation de phase dans l'étape b) et/ou dans l'étape d) des séparateurs statiques.

8. Procédé selon la revendication 1, dans lequel on réalise la réaction dans l'étape a) et/ou dans l'étape c) dans une cascade de ≤ 4 réacteurs.

9. Procédé selon la revendication 8, dans lequel le dernier réacteur dans l'étape c) est un réacteur tube.

10. Procédé selon la revendication 1, dans lequel la réaction dans l'étape a) et/ou dans l'étape c) est réalisée dans des réacteurs de polissage.

11. Procédé selon la revendication 1, dans lequel on utilise dans l'étape a) par mole de toluène ≤ 1,03 mole d'acide nitrique.

12. Procédé selon la revendication 1, dans lequel on utilise dans l'étape c) par mole de mononitrotoluène ≤ 1,03 mole d'acide nitrique.
